# EUROPEAN PATENT APPLICATION

(11) **EP 4 628 884 A1**
(43) Date of publication of application: **08.10.2025**
(21) Application number: 24168444.8
(22) Date of filing: 04.04.2024
(51) Int. Cl.: G01N 27/411

(54) **ELEMENT TO MEASURE THE OXYGEN CONTENT OF MOLTEN METALS**

(71) Applicant: Heraeus Electro-Nite International N.V., 3530 Houthalen (BE)
(72) Inventor: NEYENS, Guido, 3530 Houthalen (BE); BEX, Gert-Jan, 3530 Houthalen (BE)
(74) Representative: Heraeus IP

(57) **Abstract**

Oxygen detecting element comprising a coated pin. The coated pin comprises an electrically conductive core, which is eccentrically arranged in a coating. The coating comprises at least a two-layered coating section with an inner coating layer comprising a reference material and an outer coating layer comprising an electrolyte material. The invention further relates to an immersion sensor comprising the oxygen detecting element and a method for measuring the oxygen content of a metal melt with such an oxygen detecting element.

## Description

The invention relates to an oxygen detecting element comprising a coated pin. The coated pin comprises an electrically conductive core, which is eccentrically arranged in a coating. The invention further relates to an immersion sensor comprising the oxygen detecting element and a method for measuring the oxygen content of a metal melt with such an oxygen detecting element.

During metallurgical processes, the oxygen activity of a metal melt is one of the parameters which needs to be monitored. Determining the oxygen activity is typically done with an electrochemical sensor, comprising a solid electrolyte material, a reference material, and an electrode. The electromotive force (EMF) generated by the difference between the constant oxygen partial pressure given by the reference material and the oxygen partial pressure in the molten metal is then monitored and related to the activity or concentration of oxygen in the liquid metal. Many electrochemical sensors for the testing of such melts have shortcomings as slow response rates, high failure rates, poor reproducibility, and low sensitivity.

A type of oxygen sensor is the needle sensor, which comprises a conductive wire which serves as an electrode with at least a solid electrolyte coating and a reference material coating. These sensors suffer from a slow response time or inadequate stability for the application in the demanding environment of molten metals. An electro-chemical equilibrium between the molten metal and the oxygen sensor is necessary for an exact measurement of the EMF-value. However, an electro-chemical equilibrium only occurs if there is a thermal equilibrium between the immersion probe and its surroundings.

In order to obtain accurate measurement values, the temperature of the metal bath needs to be determined in parallel to the oxygen activity. The response time of the oxygen sensing device should ideally be faster than the response time of the temperature sensor. Mostly, thermocouples with a response time of 3 - 6 seconds are employed for this purpose.

In the needle cells as commonly used, the conductive wire is concentrically surrounded by the functional coating layers as for example disclosed in DE 2757985 A1. A reduction of the coating thickness results in a faster response time, while the stability of the coating may suffer.

JP S6179156 A discloses a needle-type oxygen concentration detecting element with a metallic wire, which comprises a coating with a conical shape to lower the response time of the device. US 5332449 A also discloses a needle sensor. The sensing device comprises a conductive wire with a uniform thickness, which is coated with an electrolyte material, a reference material and a refractory material. In order to improve the thermal responsiveness of the device, it is suggested to narrow the diameter of the conductive pin in the area without the functional coating. This configuration has been found to weaken the mechanical stability of the device.

The present invention overcomes at least parts of the problems identified in the prior art. In particular, it was an objective of the present invention to provide an oxygen detecting element with a fast response time and a high coating stability. A further aspect was to provide an oxygen detecting element which can be produced reliably and in a fast and efficient manner. It was an additional objective to provide an oxygen detecting element with a low-cost design which can be manufactured in an efficient manner.

In a different aspect, it was an objective to provide an immersion sensor with the inventive oxygen detecting element.

In a further aspect, it was an objective to provide a method for measuring the oxygen content of a metal melt with the inventive oxygen detecting element.

The present invention provides an oxygen detecting element comprising a coated pin. The coated pin comprises an electrically conductive core and a coating, wherein the coating comprises at least a two-layered coating section. The two-layered coating section comprises
(i) an inner coating layer which covers and is in direct contact with at least a part of the conductive core and comprises a reference material, and
(ii) an outer coating layer which covers and is in direct contact with at least a part of the inner coating and comprises an electrolyte material.

The electrically conductive core is arranged eccentrically in the coating.

The eccentrical arrangement of the electrically conductive core in the coating leads to a variation of the thickness of the coating around the core. While the minimal thickness seemingly determines the response time of the oxygen detecting element, the maximal thickness may contributes to the stability of the coating. Thus, the variation in the coating thicknesses results in an oxygen detecting element with a shortened response time which still provides the required mechanical strength for the intended application. Furthermore, these sensor elements can be manufactured in a more time- and cost-efficient way, providing an additional design advantage.

For certain applications, the oxygen detecting element is mounted on an immersion device to bring it in contact with the molten metal, typically comprising at least a carrier tube. These carrier tubes need to withstand the circumstances of immersion prior to their decomposition at least long enough for the measurement to be conducted, which is mostly realized by the provision of a certain amount of material. A faster response time of the oxygen detecting element allows a reduced use of material, resulting in a reduction of the costs for the immersion device. For example, the thickness of a carrier tube made of cardboard can be reduced approximately by 1 mm in diameter for every second the response time is shortened.

The object of the present invention is an oxygen detecting element comprising a coated pin with an electrically conductive core.

Examples for suitable materials for the electrically conductive core are molybdenum (Mo) and tungsten (W), especially due to their thermal properties. Preferably, the material of the conductive core comprises Mo, even more preferred the electrically conductive core consists of Mo except for unavoidable impurities. The cross-sectional area of the electrically conductive core can have any shape, preferably it is round, oval or elliptical. It is advantageous for short response times, that the maximum cross-sectional area of the electrically conductive core is in the range between 0,1 and 3 mm², especially between 0,3 and 1,5 mm².

The electrically conductive core extends longitudinally from a tip end to a mounting end. The axis extending from the tip end to the mounting end is referred to as the longitudinal axis of the electrically conductive core and/or the coated pin throughout this application. The length of the electrically conductive core is preferably in the range of 40 to 100 mm. The length of the electrically conductive core is to be understood as the length from the tip end to the mounting end. The shape of the electrically conductive core is not further restricted, it may for example be wire or needle shaped.

The tip end may have different shapes, for example it may be dome-shaped or flat.

In preferred embodiments, the electrically conductive core comprises a tapered section, the tapered section being a section comprising a cross-section which is tapered in a longitudinal direction towards the tip end. In other words, the electrically conductive core can comprise the tip end, which is a tapered end and the mounting end, and the cross-sectional area of the electrically conductive core is smaller at the tapered end. If not otherwise defined, a cross-section or cross-sectional area is the cross-section or cross-sectional area perpendicular to the longitudinal axis of the electrically conductive core along its length.

The tapered section can extend over the whole length of the electrically conductive core. It can also extend over only a part of its length; in such cases the electrically conductive core comprises at least two sections: the tapered section and a section with a constant diameter and cross-sectional area.

A tapered section has a length L_{TS}. Preferably, the tapered section extends over at least 10 % of the length of the electrically conductive core, more preferably over at least 20 %, even more preferred over at least 30 %. The length of the tapered section typically is in the range of 4 to 30 mm, preferably in the range of 8 to 20 mm.

The tapered section can have the same or a different cross-sectional shape as optionally present additional sections, for example the tapered section may have a rectangular cross-section and the other section may have a round, oval or elliptical shape. The shape of the tapered section may vary, especially depending on the production method of the electrically conductive core. The tapered section may have a radial-symmetrical geometry in relation to the central longitudinal axis of the electrically conductive core, in such cases it may for example be conical or frustoconical shaped. The tapered section may also have a geometry which is not radial symmetric in relation to the central longitudinal axis, in such cases it may for example be conical, frustoconical, prismatic or pyramidal shaped.

Preferably, the tapered section has a conical shape, in other words it has a round or oval cross-section and ends in a round or oval shaped tapered end.

The degree of tapering of the tapered section may be described by a taper angle, this angle is to be understood as the angle between the two tangents adjacent to the surface of the tapered section in the plane of the maximum cross-sectional area of the tapered section along its longitudinal axis. It has been shown to be favorable when the taper angle is smaller than 40°, even more preferred smaller than 30°, most preferred smaller than 20°. The taper angle may be in the range between 1 to 40°, preferably in the range between 3 to 30°, even more preferred in the range between 5 to 20°.

In preferred embodiments, the electrically conductive core is needle shaped, in other words it comprises a round-shaped cross-sectional area over its whole length, a tapered section with a conical shape and a tapered end with a round-shaped cross-section.

Preferably, the cross-sectional area of the tip end is smaller than 40 % of the maximum cross-sectional area of the electrically conductive core, more preferably smaller than 30 %, even more preferred smaller than 20 %. For example, the cross-sectional area of the pin end may be in the range of 0,5 % to 40 % of the maximum cross-sectional area, more preferably in the range of 2 % to 30 %, most preferred in the range of 5 % to 20 %. It has been found to be favorable for short response-times that the cross-sectional area of the pin end is between 0,01 and 1 mm², preferred between 0,02 and 0,5 mm², more preferred between 0,05 and 0,2 mm².

The coated pin comprises a coating. The coating is to be understood as all layers of material which cover the electrically conductive core.

The coating may have any cross-sectional shape perpendicular to the longitudinal axis of the conductive core, preferably, the cross-section has a round, oval or elliptical shape, even more preferred the cross-section has an elliptical shape.

The geometry of the cross-sectional area of the coating can be defined by two intersecting axis which meet at an intersection point (IP): a major axis is aligned with the maximum diameter of the cross-sectional area and has a length D_{MJ}, which corresponds to the maximum diameter of the cross-sectional area. A minor axis is perpendicular to the major axis and has a length D_{MI}. The minor axis is positioned along the largest diameter perpendicular to the major axis. In case of a circular cross-sectional area, the major axis and the minor axis are of equal length. The intersection point IP may be considered as the center of the cross-sectional area.

The electrically conductive core is arranged eccentrically in the coating. In other words, the center of the conductive core and the intersection point IP of the cross-sectional area of the coating do not coincide. An eccentric position of the center of the conductive core in the coating has surprisingly been found to have a positive effect on the response time of the oxygen detecting element and the stability of the coating.

In an eccentric configuration, the center of the conductive core is positioned with an offset to the intersection point IP along the major axis of the cross-section of the coating, thus, the coating comprises two thicknesses along the major axis, a smaller thickness Ts and a larger thickness T_{MAX}, which corresponds to the maximal thickness of the coating structure in the cross-sectional area. It is to be understood, that the thickness of the coating and the maximal thickness of the coating may vary along the length of the coated pin.

In preferred embodiments, the maximal thickness T_{MAX} is at least 5 % larger than the smaller thickness Ts, more preferred at least 8 % larger. For example, the maximal thickness T_{MAX} is 5 to 20 % larger than the smaller thickness Ts, more preferred 8 to 15 % larger.

The maximum cross sectional-area of the coated pin may be in the range between 0,5 and 7 mm², especially between 1,0 and 6 mm². The cross-sectional area of the coated pin is to be understood as the combined cross-sectional area of the electrically conductive core and the surrounding coating layers.

The coating of the coated pin comprises at least a two-layered coating section. A section of the coating is to be understood as a coating portion which comprises the same layers.

An inner coating layer covers and is in direct contact with at least a part of the electrically conductive core and an outer coating layer covers and is in direct contact with at least a part of the inner coating. In other words, no coating layer is positioned between the inner and the outer coating layer. Additional coating layers may be present on top of the outer coating layer.

The inner coating layer comprises a reference material. Preferably, the reference material comprises a metal-metal oxide mixture, for example a mixture of chromium and chromium dioxide (Cr-Cr₂O₃) or molybdenum and molybdenum oxide (Mo-MoO₂). The inner coating layer preferably has a thickness of at least 0,01 mm, more preferably of at least 0,03 mm, even more preferred of at least 0,05 mm. In this context the term "thickness" or "coating thickness" refers to the minimal thickness of the coating layer perpendicular to the longitudinal axis of the electrically conductive core. For example, the inner coating layer can have a thickness between 0,01 to 0,3 mm, more preferred between 0,03 to 0,2 mm. The thickness of the reference material coating layer may be uniform along the length of the coated pin, the thickness may also vary.

The outer coating layer comprises an electrolyte material. The electrolyte material is preferably a solid material with oxygen ion conducting activity. Preferably, the electrolyte material comprises zirconium oxide (zirconia, ZrO₂) or a stabilized zirconium oxide (stabilized zirconia). As known to the skilled person, a stabilized zirconium oxide comprises at least one kind of oxide such as magnesia (MgO), calcium oxide (CaO), yttria (Y₂O₃), ceria (CeO₂) or scandia (Sc₂O₃) solid-dissolved as a stabilizer in zirconia. The outer coating layer preferably has at least a thickness of 0,05 mm, more preferably of at least 0,1 mm, even more preferred of at least 0,15 mm. For example, the outer coating layer can have a thickness between 0,05 to 0,5 mm, more preferred between 0,1 to 0,4 mm. The thickness of the electrolyte material coating layer may be uniform along the length of the coated pin, the thickness may also vary.

The two-layered coating structure preferably covers the tip portion of the electrically conductive core, which comprises the tip end. In other words, the two-layered coating structure covers at least the tip end of the electrically conductive core. The two layered coating structure is therefore also referred to as the tip coating structure. The tip portion of the electrically conductive core is characterized in that it is covered by the tip coating structure. The tip portion of the electrically conductive core and the two-layered coating build the measuring section of the coated pin.

Preferably, the cross-sectional area of the coated pin end of the electrically conductive core is smaller than 40 % of the maximum cross-sectional area of the coated pin, more preferably smaller than 30 %, even more preferred smaller than 20 %. The coated pin end is to be understood as the coated end and the tip coating structure. A ratio of the cross-sections in this range results in a coated pin with a fast response time with a simultaneous sufficient stability. For example, the cross-sectional area of the coated pin end may be in the range of 0,5 % to 40 % of the maximum cross-sectional area of the coated pin, more preferably in the range of 2 % to 30 %, most preferred in the range of 5 % to 20 %. It has been found to be favorable for short response-times that the cross-sectional area of the coated pin end is between 0,1 and 2,5 mm², especially between 0,5 and 1,5 mm².

The tip portion has a length L_{TP}. Preferably, the tip portion extends over not more than 10 % of the length of the electrically conductive core, more preferably over not more than 5 %, even more preferred over not more than 1 %. The length of the tip portion may be in the range of 0,1 to 10 mm, more preferred in the range of 1 to 8 mm.

In cases of electrically conductive cores with a tapered section, it is preferred that the length of the tapered section of the electrically conductive core has the same length or is longer than the length of the tip portion of the electrically conductive core (L_{TS} ≥ L_{TP}). Since the measuring zone is to be found in the tapered section of the coated pin in such cases, this configuration allows a fast heating of the measuring zone and results in an especially short response time of the oxygen detecting element.

The length of the tip portion L_{TP} is preferably at least 20 % of the length of the tapered section L_{TS} of the electrically conductive core, more preferably at least 30 %, even more preferred at least 50 %.

The coating of the coated pin may comprise further coating sections. In other words, the electrically conductive core may comprise one or more portions which are covered with further coating structures and/or portions which are not coated.

For example, the coating may comprise a three-layered main coating structure (CS-M), which covers a main portion of the electrically conductive core. It is to be understood, that such a main portion of the electrically conductive core is characterized in that it is covered by the main coating structure. In such cases, the main portion is preferably located behind the tip portion in a direction from the tip end to the mounting end of the electrically conductive core.

The main portion has a length L_{MP}. Preferably, the main portion extends over more than 30 % of the length of the electrically conductive core, more preferably over more than 40 %, even more preferred over more than 50 %. The length of the main portion is typically in the range of 5 to 50 mm, preferably in the range of 10 to 40 mm.

The main coating structure may have any cross-sectional shape perpendicular to the longitudinal axis of the electrically conductive core, preferably, the cross-section has a round, oval or elliptical shape, even more preferred the cross-sectional has an elliptical shape. In other words, the main coating structure may have a constant thickness perpendicular to the longitudinal axis of the coated pin, the thickness may also vary.

In preferred embodiments, the main coating structure has a minimal thickness of at least 0,07 mm, more preferred of at least 0,12 mm. For example, the main coating structure can have a thickness between 0,07 to 0,8 mm, more preferred between 0,12 to 0,6 mm. The thickness of the main coating structure may be uniform along the length of the coated pin, the thickness may also vary.

In preferred embodiments, the main coating structure has a larger minimal thickness than the tip coating structure. Preferably, the main coating structure has a larger minimal diameter than the tip coating structure.

The main coating structure (CS-M) preferably comprises an inner coating layer, an intermediate coating layer and an outer coating layer. The inner coating layer covers and is in direct contact with at least a part of the main portion of the electrically conductive core, the intermediate coating layer covers and is in direct contact with the inner coating layer and the outer coating layer covers and is in direct contact with at least a part of the intermediate coating layer. Additional coating layers may be present on top of the outer coating layer.

The inner coating layer of the main coating structure preferably comprises a reference material. The reference material of the main coating structure may be the same as the reference material of the tip coating structure.

The inner coating layer of the main coating structure preferably has a thickness of at least 0,01 mm, more preferably of at least 0,03 mm, even more preferred of at least 0,05 mm. For example, the inner coating can have a thickness between 0,01 to 0,3 mm, more preferred between 0,03 to 0,2 mm.

Preferably, the inner coating layer of the main coating structure and the inner coating layer of the tip coating structure are sections of an inner coating layer, in other words they build a single coating layer over at least the main portion and the tip portion of the electrically conductive core. The thickness of the inner coating layer may be uniform along the length of the coated pin, the thickness may also vary.

The intermediate coating layer of the main coating structure preferably comprises a refractory material. Preferably, the refractory material comprises an oxidic material, for example an aluminum oxide like Al₂O₃, a magnesium oxide, a titanium oxide or mixtures thereof. Preferably, the refractory material comprises aluminum oxide. The intermediate coating layer preferably has at least a thickness of 0,01 mm, more preferably of at least 0,03 mm, even more preferred of at least 0,05 mm. For example, the intermediate coating can have a thickness between 0,01 to 0,3 mm, more preferred between 0,03 to 0,2 mm.

The outer coating layer of the main coating structure preferably comprises an electrolyte material. The electrolyte material of the main coating structure may be the same as the electrolyte material of the tip coating structure.

The outer coating layer of the main coating structure preferably has a thickness of at least 0,05 mm, more preferably of at least 0,1 mm, even more preferred of at least 0,15 mm. For example, the outer coating can have a thickness between 0,05 to 0,5 mm, more preferred between 0,1 to 0,4 mm.

Preferably, the outer coating layer of the main coating structure and the outer coating layer of the tip coating structure are sections of an outer coating layer, in other words they build a single coating layer over at least the main portion and the tip portion of the electrically conductive core. The thickness of the outer coating layer may be uniform along the length of the conductive pin, the thickness may also vary.

In preferred embodiments, the coating may comprise a third coating structure (CS-3), which covers a third portion of the electrically conductive core. It is to be understood, that such a third portion of the electrically conductive core is characterized in that it is covered by the third coating structure. In such cases, the third portion is preferably located behind the main portion in a direction from the tip end to the mounting end of the electrically conductive core.

Preferably, the third coating structure (CS-3) comprises an intermediate coating layer and an outer coating layer. The intermediate coating layer covers and is in direct contact with at least a part of the third portion of the electrically conductive core and the outer coating layer covers and is in direct contact with at least a part of the intermediate coating layer. In other words, no coating layer is between the intermediate and the outer coating. Additional coating layers may be present on top of the outer coating.

In preferred embodiments, the third coating structure has a minimal thickness of at least 0,06 mm, more preferred of at least 0,1 mm. For example, the third coating structure can have a thickness between 0,06 to 0,6 mm, more preferred between 0,1 to 0,5 mm. The thickness of the third coating structure may be uniform along the length of the coated pin, the thickness may also vary.

In preferred embodiments, the third coating structure has a smaller minimal thickness than the main coating structure. Preferably, the third coating structure has a smaller minimal diameter than the main coating structure.

The third portion has a length L_{3P}. Preferably, the third portion extends over not more than 10 % of the length of the electrically conductive core, more preferably over not more than 5 %, even more preferred over not more than 1 %. The length of the third portion section is typically in the range of 0,1 to 10 mm, preferably in the range of 1 to 8 mm.

The intermediate coating layer of the third coating structure may comprise a refractory material. The refractory material of the third coating structure may be the same as the refractory material of the main coating structure. The intermediate coating layer of the third coating structure preferably has at least a thickness of 0,01 mm, more preferably of at least 0,03 mm, even more preferred of at least 0,05 mm. For example, the intermediate coating can have a thickness between 0,01 to 0,3 mm, more preferred between 0,03 to 0,2 mm.

Preferably, the intermediate coating layer of the third coating structure and the intermediate coating layer of the main coating structure are sections of an intermediate coating layer, in other words they build a single coating layer over at least the third portion and the main portion of the electrically conductive core. The thickness of the intermediate coating layer may be uniform along the length of the coated pin, the thickness may also vary.

The outer coating layer of the third coating structure preferably comprises an electrolyte material. The electrolyte material of the third coating structure may be the same as the electrolyte material of the tip coating structure and/or of the main coating structure. The outer coating layer of the third coating structure preferably has a thickness of at least 0,05 mm, more preferably of at least 0,1 mm, even more preferred of at least 0,15 mm. For example, the outer coating can have a thickness between 0,05 to 0,5 mm, more preferred between 0,1 to 0,4 mm.

Preferably, the outer coating layer of the third coating structure, the outer coating layer of the main coating structure and the outer coating of the tip coating structure are sections of an outer coating layer, in other words they build a single coating layer over at least the third portion, the main portion and the tip portion of the electrically conductive core. The thickness of the outer coating layer may be uniform along the length of the coated pin, the thickness may also vary.

Preferably, the electrically conductive core comprises a mounting portion located at the mounting end which is not coated. In such cases, the electrically conductive core extends longitudinally from the mounting portion towards the main portion to the tip portion. In such cases, the mounting portion is preferably located behind the coated portion or coated portions in the direction from the tip end to the mounting end of the electrically conductive core.

The manufacturing of the inventive oxygen detecting element is typically conducted in a stepwise manner, in which the different coating layers are applied sequentially. The coatings of the coating structures may be applied by techniques known to the skilled person, for example by chemical or physical vapor deposition processes (PVD, CVD), additive manufacturing methods as 3D printing, or spray processes like plasma or flame spraying. Especially spray processes produce uniform and dense coatings. A suitable method is for example disclosed in EP 0543081 A1. During such a spray process, the object to be coated is typically moved laterally through a spray cone comprising the coating material, provided by a suitable source like a plasma or flame source, for example by means of a spray gun. In the present case, this object is the electrically conductive core. During the passage through the spray cone, the object is typically rotated to provide a homogeneous circumferential coating. To obtain a coating with a centrally positioned conductive core, this rotational movement may be centralized relative to the coating source. To obtain an eccentrical coating, this rotational movement may be eccentrical relative to the source and the lateral movement. Such an eccentrical configuration allows to coat more conductive cores at the same time, leading to a more time- and cost-efficient manufacturing process.

The manufacturing may for example comprise the following steps:
(i) providing the electrically conductive core;
(ii) coating the electrically conductive core at least partly with a reference material;
(iii) coating the electrically conductive core at least partly with an electrolyte material.

For embodiments in which the coating comprises a tip coating section and a main coating section, the manufacturing may for example comprise the following steps:
(i) providing the electrically conductive core;
(ii) coating the pin portion and the main portion of the electrically conductive core with a reference material;
(iii) masking the pin portion of the electrically conductive core;
(iv) coating at least the main portion of the electrically conductive core with a refractory material;
(v) unmasking the pin portion of the electrically conductive core;
(vi) coating at least the pin portion and the main portion with an electrolyte material.

In a second aspect, the present invention relates to an immersion sensor comprising an oxygen detecting element according to the invention.

The immersion sensor may comprise further parts, such as a counter electrode, additional measuring means like means for temperature measurement, means for mounting the oxygen detecting element, means for signal transfer, means for protecting the oxygen detecting element and/or means for immersion. Further components of an immersion sensor are known to the skilled person and are for example disclosed in US 4964736 A.

Means for temperature measurement may for example be a thermocouple as known to the skilled person. Means for temperature measurement are not mandatory for a functional immersion sensor, in cases where the temperature is needed the temperature can for example also be derived by external means.

Means for mounting the oxygen detecting element may for example be a refractory mounting material in which the oxygen detecting element can be partly embedded, preferably only an uncoated mounting portion of the electrically conductive core are embedded in such a refractory material.

Means for immersion of the immersion sensor may for example be carrier tubes, preferably made from cardboard. Means for immersion are not mandatory for a functional immersion sensor; for example, when the immersion sensor is a drop sensor. Drop-sensors and their components are known to the skilled person and are for example disclosed in EP 0997716 A1.

In a third aspect, the present invention relates to a method for measuring the oxygen content of a metal melt with an oxygen detecting element according to the invention. As known to the skilled person, the method comprises the immersion of the oxygen detecting element in the respective metal melt.

The method may for example comprise the following steps:
(i) Providing the oxygen detecting element;
(ii) immersion the oxygen detecting element in a metal melt;
(iii) measuring the oxygen content in the metal melt.

The step of measuring the oxygen content in the metal melt may comprise the measurement of the oxygen activity of the metal melt and relating this measurement to the oxygen content.

In practice the metal melt is contacted with an oxygen detecting element in accordance with the invention and the electrochemical potential of the element is measured, preferably over time. The electrochemical activity is determined by the potential expressed from the cell through lead means to an analyzing unit. By relating the electrochemical potential thus determined to electrochemical potential, which is experienced for a series of standards, the content of the oxygen within the metal melt can be determined.

The following schematic drawings show aspects of the invention for improving the understanding of the invention in connection with some exemplary illustrations. It should be understood, however, that the invention is not limited to the precise arrangements and instrumentalities shown. The elements of the drawings are not necessarily to scale relative to each other. Like reference numerals designate corresponding similar parts. Herein
- Figure 1: shows different geometries of tip-ends of electrically conductive cores.
- Figure 2: shows schematic cross-sections of electrically conductive cores suitable for the present invention.
- Figure 3: shows schematic longitudinal cross-sectional views of oxygen detecting elements according to the invention.
- Figure 4: shows schematic transverse cross-sectional views of oxygen detecting elements.
- Figure 5: shows schematic transverse cross-sectional views of the measuring zone of oxygen detecting elements.

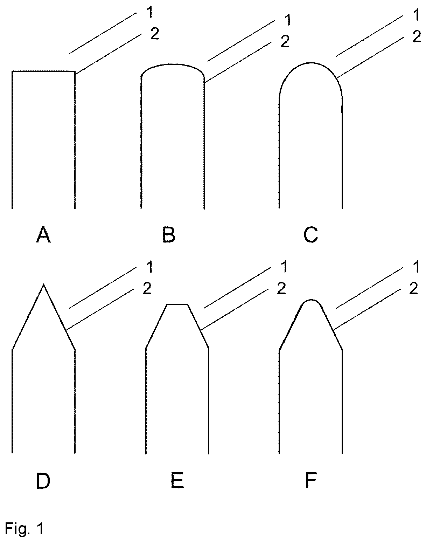

Figure 1 shows cross-sections of different geometries of tip-ends 2 of electrically conductive cores 1. The tip-ends 2 in Fig. 1 A-C are essentially flat and dome-shaped to different extends. The tip-end 2 shown in Fig. 1 A has sharp edges, wherein the edges in Fig. 1 B and C are rounded, resulting in a round (i.e. dome-shaped) tip. The tip-end 2 in Fig. 1 D is needle shaped and ends in a sharp tip. The tip-end 2 shown in Fig. 1 E has a flattened needle shape, also referred to as a frustoconical shape. The tip-end 2 illustrated in Fig. 1 F comprises a dome-shaped needle tip.

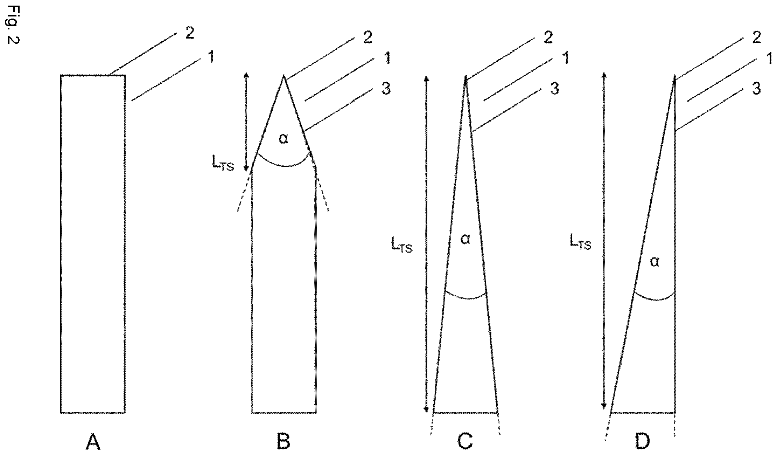

Figure 2 shows schematic longitudinal cross-sections of electrically conductive cores 1 suitable for the present invention. The core 1 of Fig. 2 A is a wire without tapered ends. The pins 1 of Fig. 2 B - D have different geometries with respect to a tapered section 3, the length of this section (L_{TS}) and the degree of tapering at their ends. It is also illustrated where the degree of tapering is to be found, indicated by the taper angle α. The taper angle is determined by the angle between two tangents adjacent to the surface of the tapered section in the plane of the maximum cross-section of the tapered section along the longitudinal axis of the pin. These tangents are indicated by dashed lines. The cores 1 of Fig. 2 B and C have a central symmetrical geometry. Fig. 2 B shows a needle shaped conductive pin 1 with a tapered section 3 which only extends over a part of the length of the pin. The tapered section 3 of the conductive core 1 shown in Fig. 2 C extends over the whole length on the pin 1. The conductive core 1 of Fig. 2 D also has a tapered section 3 over its whole length, but the geometry is not central-symmetric.

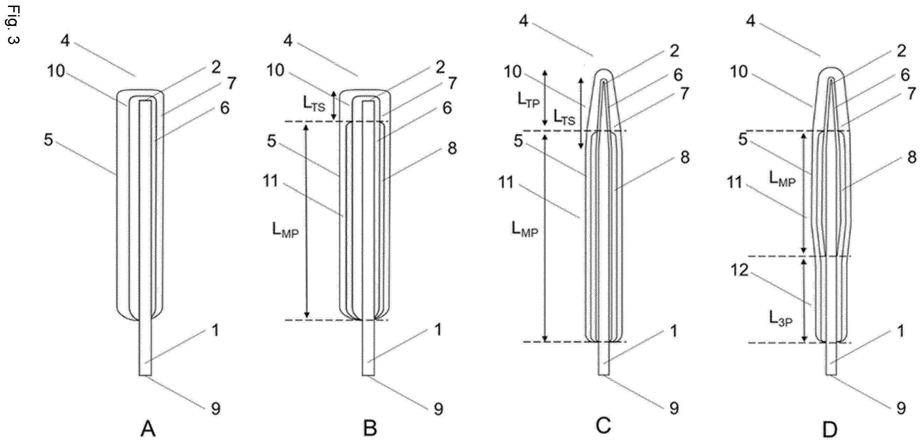

Figure 3 shows schematic longitudinal cross-sectional views of oxygen detecting elements 4 according to the invention. The conductive wire 1 of Fig. 3 A has a two-layered coating 5 with a reference material coating 6 and an electrolyte material coating 7. The end of the conductive pin 9 opposite the tip end 2 is not coated. Typically, this end is mounted in a suitable material, for example a refractory material, when the oxygen detecting element is mounted in a sensor assembly, it is therefore also referred to as the mounting end.

The coating layers which cover the conductive pin can be applied by means of a spray-process, e.g., plasma-spraying or flame-spraying, which produces a very uniform and dense coating. First, a reference material like chromium-chromium dioxide is applied to the conductive followed by a coating step with an electrolyte material like stabilized zirconium oxide.

The thicknesses of the different layers can be uniform along the length of the coated pin but they can also vary, especially when a spraying process as described is applied to manufacture the sensing element. These different layers may have the same thickness, depending on the demand and application of the oxygen detecting element, they may also vary in shape and thickness.

The electrically conductive core in Fig. 3 B comprises a coating with two portions: a first portion, the tip coating structure 10 (CS-T) covering the tip portion of the pin 1 with a length L_{TP} and a second portion, the main coating structure 11 (CS-M), covering the main portion of the pin 1 with a length L_{MP}. The tip coating structure 10 and the tip end 2 provide the measuring zone of the sensor 4 when it is in use. The main coating structure 11 comprises a three-layered structure. Additional to the two layers on the tip coating structure (6, 7), it comprises a reference material layer 8, which extends between the reference material coating 6 and the electrolyte material coating 7.

In the displayed embodiment, the electrolyte material layer 7 and the reference material layer 6 extend over the whole length of the coating structure, while the refractory material layer 8 is only present over the length of the main portion L_{MP}.

In the views of Fig. 3 A and B the conductive pins are positioned eccentrically in the coating structures. For the sake of clarity, the views of Fig. 3 C and D show the pin positioned centrally.

The needle-shaped conductive pin 1 as shown in Fig. 3 C also has a coating structure with two sections: the tip coating structure 10 (CS-T) covering the tip portion and the main coating structure 11 (CS-M), covering the main portion of the pin 1.

In comparison to the embodiment of Fig. 3 C, the coating of the embodiment illustrated in Fig. 3 D comprises an additional third coating structure 12 on a third portion of a needle-shaped conductive pin 1 with a length L_{3P}. The third coating structure 12 comprises a two-layered structure of the refractory material layer 8 and the electrolyte material layer 7. The two layers essentially comprise the same thickness.

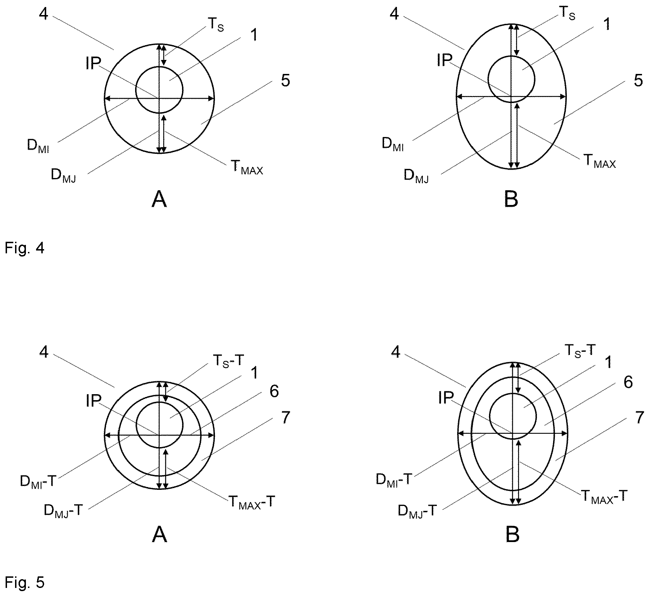

Figure 4 shows schematic transverse cross-sectional views of oxygen detecting elements 4, i.e. in a plane perpendicular to the plane as shown in Fig. 1-3. Shown is the structure of the whole coating 5 without any inner layer structure, however, the coating may comprise several layers.

Indicated are the major axis D_{MJ} and of the minor axis D_{MI} of the cross-sectional area of the coatings 5 as well as the intersection point IP of these axis, the maximal thickness of the coating structure T_{MAX} and the smaller thickness Ts along the major axis.

The coating 5 of Fig. 4 A has a circular shape. Accordingly, the length of the major axis D_{MJ} and of the minor axis D_{MI} of the cross-section are equal. The pin 1 is eccentrically positioned in the coating - the center of the pin 1 has an off-set to the intersection point IP. The coating in Fig. 4 B has an elliptical shape and the length of the major axis D_{MJ} and of the minor axis D_{MI} differ.

Figure 5 shows schematic transverse cross-sectional views of the measuring zone of oxygen detecting elements in the area of the tip coating structure with a round circular coating in Fig. 5 A and an elliptical coating in Fig. 5 B. The coatings have a two-layered structure - a reference material coating 6 directly on the pin 1 and an electrolyte material coating 7 on top of the reference material coating.

### Reference Signs

- 1: Conductive core
- 2: tip end of conductive core
- 3: tapered section of conductive core
- 4: oxygen detecting element
- 5: coating
- 6: reference material coating
- 7: electrolyte material coating
- 8: refractory material coating
- 9: mounting end of coated pin

- 10: tip coating structure (CS-T)
- 11: main coating structure (CS-M)
- 12: third coating structure (CS-3)

- L_{TS}: length of tapered section
- L_{TP}: length of tip portion
- L_{MP}: length of main portion
- L_{3P}: length of third portion
- IP: intersection point
- L_{TS}: length of tapered section
- L_{TP}: length of tip portion
- L_{MP}: length of main portion
- L_{3P}: length of third portion
- D_{MJ}: major axis of coating
- D_{MI}: minor axis of coating
- T_{MAX}: maximal thickness of coating
- Ts: smaller thickness of coating
- D_{MJ}-T: major axis of tip coating structure
- D_{MI}-T: minor axis of tip coating structure
- T_{MAX}-T: maximal thickness of tip coating structure
- Ts-T: smaller thickness of tip coating structure

- α: Taper angle

## Claims

1. An oxygen detecting element, comprising a coated pin,
the coated pin comprising an electrically conductive core and a coating,
wherein the coating comprises at least a two-layered coating section comprising
(i) an inner coating which covers and is in direct contact with at least a part of the electrically conductive core and comprises a reference material, and
(ii) an outer coating which covers and is in direct contact with at least a part of the inner coating and comprises an electrolyte material,
**characterized in that** the electrically conductive core is arranged eccentrically in the coating.

2. An oxygen detecting element according to claim 1, wherein the cross-sectional shape of the coating perpendicular to a longitudinal axis of the conductive core has a round, oval or elliptical shape.

3. An oxygen detecting element according to claim 1 or 2, wherein the coating has a minimal thickness of at least 0,06 mm.

4. An oxygen detecting element according to any of the preceding claims, wherein the coating comprises two thicknesses along a major axis of the cross-sectional area of the coating, a smaller thickness Ts and a maximal thickness T_{MAX}, wherein T_{MAX} is at least 5 % larger than T_{S}.

5. An oxygen detecting element according to any of the preceding claims, wherein the maximum cross sectional-area of the coated pin is in the range between 0,5 and 7 mm²,

6. An oxygen detecting element according to any of the preceding claims, wherein the coating comprises a three-layered main coating structure, which covers a main portion of the electrically conductive core.

7. An oxygen detecting element according to the preceding claim, wherein the coating comprises a third coating structure, which covers a third portion of the electrically conductive core.

8. An oxygen detecting element according to any of the preceding claims, wherein the electrically conductive core comprises a mounting portion located at a mounting end which is not coated.

9. An oxygen detecting element according to any of the preceding claims, wherein the electrically conductive core comprises a tapered section.

10. An oxygen detecting element according to the preceding claim, wherein the tapered section extends over at least 10 % of the length of the electrically conductive core.

11. Immersion sensor comprising an oxygen detecting element according to any of claims 1 - 10.

12. A method for measuring the oxygen content of a metal melt with an oxygen detecting element according to any of claims 1-10.
